(19) Europäisches Patentamt
European Patent Office
Office européen des brevets

(11) **EP 1 974 812 A1**

(12) **EUROPEAN PATENT APPLICATION**
published in accordance with Art. 158(3) EPC

(43) Date of publication:
**01.10.2008 Bulletin 2008/40**

(51) Int Cl.:
*B01J 29/035* (2006.01)    *C07C 1/24* (2006.01)
*C07C 11/04* (2006.01)    *C07C 11/06* (2006.01)
*C07C 11/08* (2006.01)    *C07B 61/00* (2006.01)

(21) Application number: **07706960.7**

(22) Date of filing: **18.01.2007**

(86) International application number:
**PCT/JP2007/050656**

(87) International publication number:
**WO 2007/083684 (26.07.2007 Gazette 2007/30)**

(84) Designated Contracting States:
**DE FR GB**

(30) Priority: **21.01.2006 JP 2006013259**
**29.11.2006 JP 2006321443**

(71) Applicant: **TOKYO INSTITUTE OF TECHNOLOGY**
**Tokyo 152-8550 (JP)**

(72) Inventors:
• **IWAMOTO, Masakazu**
**Yokohama-shi**
**Kanagawa 226-8503 (JP)**

• **YAMAMOTO, Takashi**
**Yokohama-shi**
**Kanagawa 226-8503 (JP)**
• **HAISHI, Teruki**
**Yokohama-shi**
**Kanagawa 226-8503 (JP)**

(74) Representative: **Kramer - Barske - Schmidtchen**
**European Patent Attorneys**
**Landsberger Strasse 300**
**80687 München (DE)**

(54) **CATALYSTS AND PROCESS FOR THE PRODUCTION OF OLEFINS WITH THE SAME**

(57)    Novel catalyst obtained by supporting one or two or more kinds of those selected from the group consisting of nickel, aluminum, manganese, iron and copper on regular meso-porous material or comprising the regular meso-porous material, and working to form one or two or more kinds of olefins by using one or two or more kinds of alcohols as starting materials. A main component of the wall constituting the regular meso-porous material is silica. The regular meso-porous material has a pore diameter in a range of 1.4 to 10 nm. One or two or more kinds of those selected from the group consisting of nickel, aluminum, manganese, iron and copper are supported on the regular meso-porous material by a template ion-exchange method. The alcohol has carbon atoms in a number in a range of 2 to 10.

EP 1 974 812 A1

**Description**

BACKGROUND OF THE INVENTION

Field of the Invention

This invention relates to novel catalyst. The invention further relates to a novel method of producing olefins by using the catalyst.

Description of the Related Art

Technologies that utilize ethyl alcohol have heretofore been reported, such as the one for producing ethylene by dehydration, the one for synthesizing gas by reforming with water vapor and the one for producing hydrocarbons.

There has been known that a variety of hydrocarbons such as ethylene, propylene, butene, etc. can be obtained at not lower than 573K if ethyl alcohol is introduced onto acid catalyst as represented by HZSM5 accompanied, however, by the occurrence of problems, such as formation of a multiplicity of products over a wide range, formation of cokes, short life of the catalyst in which the catalytic activity does not last long but is deteriorated conspicuously, and the product selectivity varies with the passage of time, that must be solved. The presence of water suppresses the formation of cokes to some extent. Still, however, it is not possible to avoid the formation of aromatic compounds or to avoid the aging of the products (see, for example, non-patent documents 1, 2 and 3).

There has been, further, reported that ethylene, acetaldehyde, butadiene, butanol and diethyl ether are formed at 623 to 823K if vapor of ethyl alcohol is introduced onto catalyst obtained by supporting aluminum or nickel on calcium phosphate. However, formation of olefins other than the ethylene has not yet been reported (see, for example, patent document 1).

The present inventors are disclosing the technical contents related to the present invention (see, for example, non-patent documents 4 and 5).

Patent document 1: JP-A-11-217343
Non-patent document 1: C.W. Ingram, R.J. Lancashire, Catal. Lett., 31, 395(1995).
Non-patent document 2: A.K. TaLuKdar, K.G. Bhattacharyya, S. SivasanKar, Appl. CataL. A, 148, 357(1997). Non-patent document 3: A.T. Aguayo, A.G. Gayubo, A.M. Tarrio, A. Atutxa, J. BiLbao, J. Chem. Tech. Biotech., 77,211 (2002).
Non-patent document 4: K. Kasai, T. Haishi, T. Yamamoto, M. Iwamoto, 98th Forum on Catalysts, 3105(2006). Non-patent document 5: K. Kasai, T. Haishi, T. Yamamoto, M. Iwamoto, 36th Forum on Petroleum and Petrochemistry, 2A19(2006).

Though several examples were introduced above, it has now been urged to develop a technology for producing olefins from alcohols highly actively, highly selectively and continuously.

SUMMARY OF THE INVENTION

This invention was accomplished in view of the above problems and has an object of providing novel catalyst. The invention, further, has another object of providing a novel method of producing olefins by using the above catalyst.

In order to achieve the above objects by solving the above problems, the present invention provides catalyst obtained by supporting one or two or more kinds of those selected from the group consisting of nickel, aluminum, manganese, iron and copper on regular meso-porous material or comprising the regular meso-porous material, and works to form one or two or more kinds of olefins by using one or two or more kinds of alcohols as starting materials.

Here, though not limited, it is desired that a main component of the wall constituting the regular meso-porous material is silica. Further, though not limited, it is desired that the regular meso-porous material has a pore diameter in a range of 1.4 to 10 nm. Further, though not limited, it is desired that one or two or more kinds of those selected from the group consisting of nickel, aluminum, manganese, iron and copper are supported on the regular meso-porous material by a template ion-exchange method. Further, though not limited, it is desired that nickel is supported on the regular meso-porous material. Further, though not limited, it is desired that the alcohol has carbon atoms in a number in a range of 2 to 10. Further, though not limited, it is desired that the alcohol is ethyl alcohol.

A method of producing olefins of the invention comprises forming one or two or more kinds of olefins by using one or two or more kinds of alcohols as starting materials in the presence of catalyst obtained by supporting one or two or more kinds of those selected from the group consisting of nickel, aluminum, manganese, iron and copper on regular meso-porous material or comprising the regular meso-porous material.

Here, though not limited, it is desired that a main component of the wall constituting the regular meso-porous material is silica. Further, though not limited, it is desired that the regular meso-porous material has a pore diameter in a range of 1.4 to 10 nm. Further, though not limited, it is desired that one or two or more kinds of those selected from the group consisting of nickel, aluminum, manganese, iron and copper are supported on the regular meso-porous material by a template ion-exchange method. Further, though not limited, it is desired that nickel is supported on the regular meso-porous material. Further, though not limited, it is desired that the alcohol has carbon atoms in a number in a range of 2 to 10. Further, though not limited, it is desired that the alcohol is ethyl alcohol.

The invention exhibits the effects as described below.

The invention provides novel catalyst, i.e., catalyst obtained by supporting one or two or more kinds of those selected from the group consisting of nickel, aluminum, manganese, iron and copper on regular meso-porous material or comprising the regular meso-porous material, and works to form one or two or more kinds of olefins by using one or two or more kinds of alcohols as starting materials.

The invention provides a novel method of producing olefins, comprising forming one or two or more kinds of olefins by using one or two or more kinds of alcohols as starting materials in the presence of catalyst obtained by supporting one or two or more kinds of those selected from the group consisting of nickel, aluminum, manganese, iron and copper on regular meso-porous material or comprising the regular meso-porous material.

DETAILED DESCRIPTION OF THE INVENTION

The catalyst of the invention and the method of producing olefins by using the catalyst of the invention will now be described.

The catalyst of the invention includes two kinds of catalysts.

First catalyst will now be described.

The first catalyst is obtained by supporting nickel on regular meso-porous material.

The regular meso-porous material is used as a support component of the catalyst. The regular meso-porous material is inorganic or inorganic/organic composite solid material having regular nano pores. Particularly preferably, regular meso-porous material is used in which silica is a main component of the wall that constitutes the regular meso-porous material.

It is desired that the regular meso-porous material has a pore diameter in a range of 1.4 to 10 nm. If the pore diameter is not smaller than 1.4 nm, an advantage is obtained in that the reacted molecules and the formed molecules can be easily diffused. If the pore diameter is not larger than 10 nm, an advantage is obtained in that the effect of pores is efficiently exhibited.

Though there is no particular limitation on the method of synthesizing the regular meso-porous material which is support, a known synthesizing method can be employed by using quaternary ammonium salt having a higher alkyl group with not less than 8 carbon atoms as a template and using silica precursor as starting material.

As the silica precursor, there can be used amorphous silica such as colloidal silica, silica gel or fumed silica; alkali silicate such as sodium silicate or potassium silicate; or alkoxide of silicon, such as tetramethyl orthosilicate or tetraethyl orthosilicate, in one kind or being mixed together.

Though there is no particular limitation on the kind of the template used for the synthesis of the regular meso-porous material, it is particularly desired to use cationic surfactant of the type of alkyltrimethylammonium halide represented by the general formula $CH_3(CH_2)_nN(CH_3)_3 \cdot X$ (n is an integer of 7 to 21 and X is halogen ion or hydroxide ion). Concretely, there can be exemplified n-octyltrimethylammonium bromide, n-decyltrimethylammonium bromide, n-dodecyltrimethylammonium bromide, n-tetradecyltrimethylammonium bromide and n-octadecyltrimethylammonium bromide.

There is no particular limitation on the method of supporting nickel on the support and a known method can be used, such as imbibition method, gaseous phase vapor deposition method or support complex decomposition method. In particular, a template ion-exchange method is desired exchanging nickel ions with template ions in aqueous medium without sintering or removing the templates occluded in the pores that are synthesizing the regular meso-porous material.

It is desired that the amount of nickel supported by the regular meso-porous material is such that the atomic ratio Si/Ni is 500 to 5 based on the silica that constitutes the wall. It is further desired that the atomic ratio Si/Ni is 100 to 15.

If the atomic ratio Si/Ni is not smaller than 5, it is allowed to suppress the formation of fine nickel oxide particles having a low catalytic activity. If the atomic ratio Si/Ni is not smaller than 15, this effect becomes more conspicuous.

If the atomic ratio Si/Ni is not larger than 500, highly dispersed nickel can be supported to a sufficient degree. If the atomic ratio Si/Ni is not larger than 100, the effect becomes more conspicuous.

The template ion exchange can be effected by bringing the regular meso-porous material having templates occluded in the pores into contact with aqueous solution containing inorganic acid salt or organic acid salt of nickel. As a source of nickel, there can be exemplified nickel acetate, nickel chloride, nickel bromide, nickel sulfate, nickel oxide, nickel hydroxide or nickel nitrate. These nickel compounds can be used in a single kind or being mixed together in two or more kinds. Among them, it is desired to use the nickel nitrate from the standpoint of easy handling and the degree of solubility in water.

The regular meso-porous material supporting nickel by the template ion-exchange method is desirably heat-treated in an atmosphere where oxygen is present in order to burn and remove the residual templates. The heat treatment is conducted preferably at 200 to 800°C and, more preferably, at 300 to 600°C.

If the heat-treating temperature is not lower than 200°C, an advantage is obtained in that the burning of templates is accelerated. If the heat-treating temperature is not lower than 300°C, the effect becomes more conspicuous.

If the heat-treating temperature is not higher than 800°C, an advantage is obtained in that the silica constituting the pore walls is prevented from being collapsed. If the heat-treating temperature is not higher than 600°C, the effect becomes more conspicuous.

The metal supported by the regular meso-porous material is not limited to nickel. There can be used one or two or more kinds of those selected from the group consisting of nickel, aluminum, manganese, iron and copper.

Next, described below is a method of producing olefins by using the first catalyst.

The method of producing olefins comprises forming one or two or more kinds of olefins by using one or two or more kinds of alcohols as starting materials in the presence of catalyst obtained by supporting one or two or more kinds of those selected from the group consisting of nickel, aluminum, manganese, iron and copper on the regular meso-porous material.

It is desired that the alcohol has carbon atoms in a number in a range of 2 to 10. Concrete examples of the alcohol include ethyl alcohol, propyl alcohol, butyl alcohol and cyclohexyl alcohol.

The reaction for forming olefin is conducted in the gaseous phase. That is, the reaction is conducted by introducing starting gas of alcohol vapor into a reactor charged with the catalyst. The starting gas may, further, contain saturated hydrocarbons such as methane, ethane, n-butane or the like. The starting gas is directly introduced into the reactor or is introduced therein being diluted with inert gas such as nitrogen, helium, argon or carbonic acid gas.

It is desired that the alcohol partial pressure is in a range of 1.0 to 50%. If the alcohol partial pressure is not smaller than 1.0%, an advantage is obtained in that the conversion of alcohol and the selectivity into olefin are enhanced. If the alcohol partial pressure is not larger than 50%, an advantage is obtained in that the reaction gas can be easily fed and the product can be easily separated.

As a reaction condition, the reaction temperature is in a range of, preferably, 100 to 600°C and, more preferably, 350 to 450°C.

If the reaction temperature is not lower than 100°C, an advantage is obtained in that the reaction rate and the reactivity are enhanced. If the reaction temperature is not lower than 350°C, the effect becomes more conspicuous.

If the reaction temperature is not higher than 600°C, an advantage is obtained in that the activity of the catalyst is prevented from being deteriorated. If the reaction temperature is not higher than 450°C, the effect becomes more conspicuous.

The pressure condition can be suitably selected out of a wide range of from normal pressure up to a high pressure. Usually, however, the pressure is from normal pressure through up to about 1.0 MPa.

The time in which the starting gas comes in contact with the catalyst is preferably in a range of 0.001 to 10 g - catalyst· sec/cc - starting gas and, more preferably, 0.01 to 10 g - catalyst· sec/cc - starting gas. If the contacting time is not smaller than 0.001 g - catalyst· sec/cc - starting gas, an advantage is obtained in that the conversion of alcohol increases and the selectivity of olefin increases. If the contacting time is not smaller than 0.01 g - catalyst· sec/cc - starting gas, the effect becomes more conspicuous. If the contacting time is not larger than 10 g - catalyst· sec/cc - starting gas, an advantage is obtained in that the side reaction such as polymerization is suppressed.

If the starting gas contains water, it is desired that the amount of water fed into the starting gas is not larger than 5.5% by volume. If the amount of water fed into the starting gas is not larger than 5.5% by volume, an advantage is obtained in that the conversion of the starting alcohol does not decrease.

Olefins which are the reaction products may include ethylene, propylene, 1-butene, 2-butene, pentene and hexene.

In this invention, first, regular meso-porous catalyst supporting nickel is prepared by the template ion-exchange method. A fixed-bed gas phase flow reaction apparatus is charged with the catalyst, and nitrogen gas containing ethyl alcohol vapor is flown to continuously obtain lower olefins such as ethylene, propylene and butene highly selectively and in high yields.

It has been known that the butene and propylene can be selectively produced from the ethylene if the regular meso-porous material supporting nickel is used as catalyst (JP-A-2003-326169, leaflet of International Laid-Open No. 2005/023420). It is considered that the reaction proceeds through the formation of 1-butene by the dimerization of ethylene, formation of 2-butene by the isomerization of 1-butene, and metathesis of the formed 2-butene and ethylene. In the elementary process of reaction, it is considered that the butene isomerization reaction proceeds due to the function of the solid acid on the catalyst. On the other hand, it has been known that the dehydration reaction of the ethyl alcohol is catalyzed with acid. It is, therefore, considered that if the ethyl alcohol is introduced onto the catalyst comprising the regular meso-porous material supporting nickel, ethylene is selectively formed from the ethyl alcohol, and the formed ethylene is successively converted into butene and propylene.

It has been urged to develop a technology for effectively utilizing ethyl alcohol which is biomass product from the standpoint of developing resources to substitute for the petroleum, diversifying primary energy sources and reducing carbon dioxide. It is considered that the bioethanol can be produced by large-scale cultivation in South America and in Southeast Asia, and can be supplied in large amounts and inexpensively. If the bioethanol can be utilized as a resource to substitute for the petroleum, it is expected that a petrochemical industry can be created without relying upon the fossil resources. The present invention makes it possible to highly selectively and continuously produce lower olefins such as ethylene, propylene and butene having industrially high additional values from the ethyl alcohol.

According to the best mode for carrying out the invention, therefore, there is provided novel catalyst obtained by supporting one or two or more kinds of those selected from the group consisting of nickel, aluminum, manganese, iron and copper on regular meso-porous material, and works to form one or two or more kinds of olefins by using one or two or more kinds of alcohols as starting materials.

According to the best mode for carrying out the invention, further, there is provided a novel method of producing olefins comprising forming one or two or more kinds of olefins by using one or two or more kinds of alcohols as starting materials in the presence of catalyst obtained by supporting one or two or more kinds of those selected from the group consisting of nickel, aluminum, manganese, iron and copper on the regular meso-porous material.

Here, it should be noted that the invention is not limited to the above best mode for carrying out the invention only but can be further varied and modified in various ways without departing from the spirit and scope of the invention, as a matter of course.

Next, second catalyst will be described.
The second catalyst comprises the regular meso-porous material.

The regular meso-porous material is inorganic or inorganic/organic composite solid material having regular nano pores. Particularly preferably, regular meso-porous material is used in which silica is a main component of the wall that constitutes the regular meso-porous material.

It is desired that the regular meso-porous material has a pore diameter in a range of 1.4 to 10 nm. If the pore diameter is not smaller than 1.4 nm, an advantage is obtained in that the reacted molecules and the formed molecules can be easily diffused. If the pore diameter is not larger than 10 nm, an advantage is obtained in that the effect of pores is efficiently exhibited.

Though there is no particular limitation on the method of synthesizing the regular meso-porous material which is support, a known synthesizing method can be employed by using quaternary ammonium salt having a higher alkyl group with not less than 8 carbon atoms as a template and using a silica precursor as starting material.

As the silica precursor, there can be used amorphous silica such as colloidal silica, silica gel or fumed silica; alkali silicate such as sodium silicate or potassium silicate; or alkoxide of silicon, such as tetramethyl orthosilicate or tetraethyl orthosilicate, in one kind or being mixed together.

Though there is no particular limitation on the kind of the template used for the synthesis of the regular meso-porous material, it is particularly desired to use cationic surfactant of the type of alkyltrimethylammonium halide represented by the general formula $CH_3(CH_2)_nN(CH_3)_3 \cdot X$ (n is an integer of 7 to 21 and X is halogen ion or hydroxide ion). Concretely, there can be exemplified n-octyltrimethylammonium bromide, n-decyltrimethylammonium bromide, n-dodecyltrimethylammonium bromide, n-tetradecyltrimethylammonium bromide and n-octadecyltrimethylammonium bromide.

In order to remove the residual templates by burning, the regular meso-porous material is, preferably, heat-treated in an atmosphere where oxygen is present. The heat treatment is conducted at, preferably, 200 to 800°C and, more preferably, 300 to 600°C.

If the heat-treating temperature is not lower than 200°C, an advantage is obtained in that the burning of templates is accelerated. If the heat-treating temperature is not lower than 300°C, the effect becomes more conspicuous.

If the heat-treating temperature is not higher than 800°C, an advantage is obtained in that the silica constituting the pore walls is prevented from being collapsed. If the heat-treating temperature is not higher than 600°C, the effect becomes more conspicuous.

Next, described below is a method of producing olefins by using the second catalyst.

The method of producing olefins comprises forming one or two or more kinds of olefins by using one or two or more kinds of alcohols as starting materials in the presence of catalyst comprising the regular meso-porous material.

It is desired that the alcohol has carbon atoms in a number in a range of 2 to 10. Concrete examples of the alcohol include ethyl alcohol, propyl alcohol, butyl alcohol and cyclohexyl alcohol.

The reaction for forming olefins is conducted in the gaseous phase. That is, the reaction is conducted by introducing starting gas of alcohol vapor into a reactor charged with the catalyst. The starting gas may, further, contain saturated hydrocarbons such as methane, ethane, n-butane or the like. The starting gas is directly introduced into the reactor or is introduced therein being diluted with inert gas such as nitrogen, helium, argon or carbonic acid gas.

It is desired that the alcohol partial pressure is in a range of 1.0 to 50%. If the alcohol partial pressure is not smaller than 1.0%, an advantage is obtained in that the conversion of alcohol and the selectivity into olefins are enhanced. If the alcohol partial pressure is not larger than 50%, an advantage is obtained in that the reaction gas can be easily fed and the product can be easily separated.

As a reaction condition, the reaction temperature is in a range of, preferably, 100 to 600°C and, more preferably, 250 to 450°C.

If the reaction temperature is not lower than 100°C, an advantage is obtained in that the reaction rate and the reactivity are enhanced. If the reaction temperature is not lower than 250°C, the effect becomes more conspicuous.

If the reaction temperature is not higher than 600°C, an advantage is obtained in that the activity of the catalyst is prevented from being deteriorated. If the reaction temperature is not higher than 450°C, the effect becomes more conspicuous.

The pressure condition can be suitably selected out of a wide range of from normal pressure up to a high pressure. Usually, however, the pressure is from normal pressure through up to about 1.0 MPa.

The time in which the starting gas comes in contact with the catalyst is preferably in a range of 0.001 to 10 g - catalyst· sec/cc - starting gas and, more preferably, 0.01 to 10 g - catalyst· sec/cc - starting gas. If the contacting time is not smaller than 0.001 g - catalyst· sec/cc - starting gas, an advantage is obtained in that the conversion of alcohol increases and the selectivity of olefins increases. If the contacting time is not smaller than 0.01 g - catalyst· sec/cc - starting gas, the effect becomes more conspicuous. If the contacting time is not larger than 10 g - catalyst· sec/cc - starting gas, an advantage is obtained in that the side reaction such as polymerization is suppressed.

If the starting gas contains water, it is desired that the amount of water fed into the starting gas is not larger than 5.5% by volume. If the amount of water fed into the starting gas is not larger than 5.5% by volume, an advantage is obtained in that the conversion of the starting alcohol does not decrease.

Olefins which are the reaction products may include ethylene, propylene, 1-butene, 2-butene, pentene and hexene.

According to the best mode for carrying out the invention, therefore, there is provided novel catalyst which comprises regular meso-porous material, and works to form one or two or more kinds of olefins by using one or two or more kinds of alcohols as starting materials.

According to the best mode for carrying out the invention, further, there is provided a novel method of producing olefins comprising forming one or two or more kinds of olefins by using one or two or more kinds of alcohols as starting materials in the presence of catalyst which comprises the regular meso-porous material.

Here, it should be noted that the invention is not limited to the above best mode for carrying out the invention only but can be further varied and modified in various ways without departing from the spirit and scope of the invention, as a matter of course.

EXAMPLES

The invention will now be concretely described by way of Examples to which only, however, the invention is in no way limited, as a matter of course.

Reference Example 1 (Synthesis of C12-MCM-41).

225.0 Grams n-dodecyltrimethylammonium bromide and 641.4 g of ion-exchanged water were introduced into a 2-liter fluorine-contained resin beaker, and were stirred in a water bath of 313K until homogeneous solution was obtained. To this solution were added solution obtained by dissolving 11.6 g of sodium hydroxide in 125.5 g of ion-exchanged water and 306.3 g of Snowtex 20 (manufactured by Nissan Kagaku Co.) alternately and in small amounts each time, and the mixture was stirred for 2 hours. The mixed solution was transferred into a 2-liter autoclave that has a fluorine-contained resin insertion tube, and was sealed and left to stand still at 413K for 48 hours. Thereafter, the autoclave was cooled, and the content was taken out and to separate the solid component by filtration by means of suction. The solid component was washed with 2 liters of ion-exchanged water and was dried at 353K. The yield of the thus obtained solid component (C12-MCM-41) was 84.2 g. The powder X-ray diffraction measurement of the sample exhibited diffraction peaks at $2\theta$ = 2.52 degrees, 4.31 degrees and 4.96 degrees, from which it was learned that the sample possessed a hexagonal structure. The solid component was further heated up to 873K at a rate of 5 degrees/min., and was fired at this temperature for 6 hours in the air. Thereafter, the solid component was measured for its surface area and porous size by a nitrogen adsorption method to be 1014 $m^2$/g and 1.12 nm, respectively.

Example 1 (Preparation of catalyst A).

3 Grams of the unfired C12-MCM-41 obtained in Reference Example 1 were weighed out into a fluorine-contained resin container and were dispersed in 30 g of ion-exchanged water. On the other hand, 0.784 g of nickel nitrate was dissolved in 30 mL of ion-exchanged water, and the solution was transferred into the container in which the C12-MCM-41 has been dispersed and was vigorously stirred at room temperature for one hour. Here, the atomic ratio Si/Ni of silicon contained in the C12-MCM-41 and nickel that was added was 10. Thereafter, the container was wrapped, submerged in a water bath that has been maintained at 353K and was left to stand still for 20 hours. The solution was cooled down to room temperature, and the solid component was separated by filtration by means of suction. The solid component was washed with 300 mL of ion-exchanged water and was dried overnight at 353K. The obtained solid component was finely ground down in a mortar, thinly spread on a magnetic dish, introduced into an electric furnace, heated up to 773K at a rate of 5K/min., and was fired at this temperature for 6 hours. The thus obtained catalyst was analyzed by an ICP emission spectral method to find that the atomic ratio Si/Ni was 14.4. The surface area was 733 $m^2$/g.

Example 2 (Preparation of catalyst B).

The procedure was conducted in the same manner as in Example 1 but setting the atomic ratio Si/Ni of silicon contained in the C12-MCM-41 and nickel to be 20. The atomic ratio Si/Ni of the obtained catalyst was 23.0 and the surface area was 960 $m^2$/g.

Example 3 (Preparation of catalyst C).

The procedure was conducted in the same manner as in Example 1 but setting the atomic ratio Si/Ni of silicon contained in the C12-MCM-41 and nickel to be 20. The atomic ratio Si/Ni of the obtained catalyst was 23.7 and the surface area was 876 $m^2$/g.

Example 4 (Preparation of catalyst D).

The procedure was conducted in the same manner as in Example 1 but setting the atomic ratio Si/Ni of silicon contained in the C12-MCM-41 and nickel to be 50. The atomic ratio Si/Ni of the obtained catalyst was 63.0 and the surface area was 988 $m^2$/g.

Example 5 (Preparation of catalyst E).

The procedure was conducted in the same manner as in Example 1 but setting the atomic ratio Si/Ni of silicon contained in the C12-MCM-41 and nickel to be 100. The atomic ratio Si/Ni of the obtained catalyst was 102 and the surface area was 1038 $m^2$/g.

Example 6.

Quartz wool was packed on the bottom of a heat resistant glass reactor of an inner diameter of 10 mm, and a predetermined amount of the catalyst was charged thereon. A fine glass tube for inserting a thermoelectric couple was put in the center of the reaction tube to measure the temperature, and the height of the quartz wool was so adjusted that a tip of the thermoelectric couple was positioned at the center. The reaction apparatus was constituted by a mass flow controller, a micro feeder, a reaction tube and a gas chromatography (with a hydrogen flame detector) for analysis. The reaction gas was the one obtained by introducing the ethyl alcohol through the micro feeder into carrier gas of which the flow rate was controlled by the mass flow meter, and was introduced from above the catalyst layer so as to flow downward thereof to be brought to a gas sampler for gas chromatography. The reaction tube was heated by an electric furnace so that the catalyst layer assumed a predetermined temperature. Prior to the reaction, the catalyst was pre-treated by flowing nitrogen gas at a rate of 50 mL/min. at 673K for 2 hours. The reaction was conducted by feeding mixed gas (containing 5.5% by volume of ethyl alcohol) of ethyl alcohol and nitrogen at a rate of 10 mL/min. The pressure was 0.1 MPa.

Table 1 shows the results of the thus conducted reaction. In Table 1, "butene" stands for a mixture of 1-butene and 2-butene, "Ethanol conversion (%)" stands for a percentage of the mol number of ethyl alcohol that has reacted to the mol number of ethyl alcohol before reacted, and "Selectivity coefficient (%)" stands for a percentage of the mol number of the component calculated as the ethyl alcohol to the mol number of the reacted ethyl alcohol. The same also holds true in Tables 2 to 5 appearing later.

Table 1

Table 1

| Experiment No. | Catalyst | Amount of catalyst (g) | Temp. (K) | Ethanol conversion (%) | Methylene selectivity coefficient (%) | Propylene selectivity coefficient (%) | Butene selectivity coefficient (%) | Diethyl ether selectivity coefficient (%) | Acetaldehyde selectivity coefficient (%) |
|---|---|---|---|---|---|---|---|---|---|
| 1 | B | 0.2 | 473 | 7.8 | 2.3 | 0.0 | 0.0 | 50.0 | 0.0 |
| 2 | B | 0.2 | 523 | 34.4 | 13.8 | 1.5 | 0.1 | 54.9 | 7.3 |
| 3 | B | 0.2 | 573 | 71.8 | 56.5 | 6.2 | 3.8 | 13.8 | 7.1 |
| 4 | B | 0.2 | 623 | 100 | 67.0 | 11.2 | 9.2 | 0.0 | 7.8 |
| 5 | B | 0.2 | 673 | 100 | 67.0 | 15.1 | 5.2 | 0.0 | 7.3 |
| 6 | B | 0.2 | 723 | 100 | 63.7 | 16.3 | 2.9 | 0.0 | 5.6 |

Up to 523K, the main product was diethyl ether that was produced by the intermolecular dehydration reaction. Over 623K, the main product was ethylene that was produced by the intermolecular dehydration reaction, and the yield of olefin was not smaller than 80%. As the reaction temperature increased, the selectivity coefficients of ethylene and butene decreased while the selectivity coefficient of propylene increased. In Experiment No. 5, the reaction was continued for 10 hours but there was no change in the activity and selectivity.

Example 10.

The solid component (unfired) C12-MCM-41 obtained in Reference Example 1 was finely ground down in a mortar, thinly spread on a magnetic dish, introduced into an electric furnace, heated up to 773K at a rate of 5 K/min., and was fired at this temperature for 6 hours.

Next, the effect of the C12-MCM-41 without supporting nickel was examined in producing olefin from the ethyl alcohol. The experimental conditions were the same as those of Example 6 but using the C12-MCM-41 as catalyst. The results were as shown in Table 2. Over 623K, ethylene was formed nearly quantitatively. From a comparison with Table 1, it was obvious that the addition of nickel ions helped form propylene and butene.

Table 2

Table 2

| Experiment No. | Catalyst | Amount of catalyst (g) | Temp. (K) | Ethanol conversion (%) | Ethylene selectivity coefficient (%) | Propylene selectivity coefficient (%) | Butene selectivity coefficient (%) | Diethyl ether selectivity coefficient (%) | Acetaldehyde selectivity coefficient (%) |
|---|---|---|---|---|---|---|---|---|---|
| 7 | C12-MCM-41 | 0.2 | 523 | 24.2 | 13.8 | 0.0 | 0.0 | 59.1 | 0.0 |
| 8 | C12-MCM-41 | 0.2 | 573 | 58.7 | 65.7 | 0.0 | 0.0 | 24.8 | 0.0 |
| 9 | C12-MCM-41 | 0.2 | 623 | 96.3 | 99.0 | 0.2 | 0.0 | 0.0 | 0.4 |
| 10 | C12-MCM-41 | 0.2 | 673 | 100 | 99.0 | 0.3 | 0.1 | 0.0 | 1.1 |

Example 7.

The effect of the supported amount of nickel upon the catalytic activity was examined. The experimental conditions were the same as those of Example 6. The results were as shown in Table 3.

Table 3

Table 3

| Experiment No. | Catalyst | Amount of catalyst (g) | Temp. (K) | Ethanol conversion (%) | Ethylene selectivity coefficient (%) | Propylene selectivity coefficient (%) | Butene selectivity coefficient (%) | Diethyl ether selectivity coefficient (%) | Acetaldehyde selectivity coefficient (%) |
|---|---|---|---|---|---|---|---|---|---|
| 11 | A | 0.2 | 673 | 100 | 66.5 | 15.3 | 4.9 | 0.0 | 3.5 |
| 5 | B | 0.2 | 673 | 100 | 67.0 | 15.1 | 5.2 | 0.0 | 7.3 |
| 12 | C | 0.2 | 673 | 100 | 66.7 | 15.5 | 5.3 | 0.0 | 3.8 |
| 13 | D | 0.2 | 673 | 100 | 64.1 | 14.1 | 5.2 | 0.0 | 5.3 |
| 14 | E | 0.2 | 673 | 100 | 70.8 | 11.5 | 4.6 | 0.0 | 5.1 |

In experiment No. 14 containing nickel in the least amount, the propylene selectivity coefficient was slightly low but the ethylene selectivity coefficient was high. Within the range of this experiment, neither the activity nor the product selectivity greatly varied depending upon the supported amount.

Example 8.

The effect of the charged amount of the catalyst upon the catalytic activity was examined. The experimental conditions were the same as those of Example 6. The time in which the starting gas contacted the catalyst was 0.3 to 2.4 g - catalyst· sec/cc - starting gas. The results were as shown in Table 4.

Table 4

Table 4

| Experiment No. | Catalyst | Amount of catalyst (g) | Temp. (K) | Ethanol conversion (%) | Ethylene selectivity coefficient (%) | Propylene selectivity coefficient (%) | Butene selectivity coefficient (%) | Diethyl ether selectivity coefficient (%) | Acetaldehyde selectivity coefficient (%) |
|---|---|---|---|---|---|---|---|---|---|
| 15 | C | 0.05 | 673 | 100 | 72.8 | 8.4 | 4.2 | 0.0 | 11.0 |
| 16 | C | 0.1 | 673 | 100 | 71.0 | 11.7 | 4.5 | 0.0 | 9.2 |
| 12 | C | 0.2 | 673 | 100 | 66.7 | 15.5 | 5.3 | 0.0 | 3.8 |
| 17 | C | 0.4 | 673 | 100 | 55.5 | 18.1 | 5.2 | 0.0 | 0.8 |

As the amount of catalyst increased, the ethylene selectivity coefficient decreased while the propylene selectivity coefficient increased. This was because propylene was successively formed via ethylene. The acetaldehyde selectivity coefficient decreased with an increase in the amount of the catalyst accompanied, however, by an increase in the lack of carbon balance (amount of the starting ethyl alcohol converted into something that was unknown). It was, therefore, considered that the aldehyde has underwent the polymerization reaction and has changed into components that could not be detected by gas chromatography.

Example 9.

The effect of the ethanol partial pressure upon the product selectivity was examined. The experimental conditions were the same as those of Example 6 but varying the amount of ethyl alcohol introduced by using the micro feeder. The results were as shown in Table 5.

Table 5

Table 5

| Experiment No. | Catalyst | Ethanol partial pressure (%) | Amount of catalyst (g) | Temp. (K) | Ethanol conversion (%) | Ethylene selectivity coefficient (%) | Propylene selectivity coefficient (%) | Butene selectivity coefficient (%) | Diethyl ether selectivity coefficient (%) | Acetaldehyde selectivity coefficient (%) |
|---|---|---|---|---|---|---|---|---|---|---|
| 18 | B | 2.8 | 0.2 | 673 | 100 | 60.7 | 14.5 | 3.0 | 0.0 | 8.0 |
| 5 | B | 5.5 | 0.2 | 673 | 100 | 67.0 | 15.1 | 5.2 | 0.0 | 7.3 |
| 19 | B | 12.6 | 0.2 | 673 | 100 | 60.0 | 12.5 | 7.3 | 0.0 | 8.1 |

As the ethanol partial pressure increased, the butene selectivity coefficient slightly increased without, however, any conspicuous change in the product selectivity. Even in experiment No. 19 having the highest ethanol partial pressure, the activity did not almost change up to the reaction time of 10 hours.

Example 11.

The effect of the amount of catalyst C12-MCM-41 was examined. The experimental conditions were the same as those of Example 10 but increasing the amount of the catalyst from 0.2 g to 0.5 g and varying the reaction temperature to 523 to 723 K. Upon increasing the amount of the catalyst, the selectivity coefficient of ethylene which was the object product increased when the reaction temperature was 523 to 573K.
Table 6

Table 6

| Experiment No. | Catalyst | Amount of catalyst (g) | Temp. (K) | Ethanol conversion (%) | Ethylene selectivity coefficient (%) | Propylene selectivity coefficient (%) | Butene selectivity coefficient (%) | Diethyl ether selectivity coefficient (%) | Acetaldehyde selectivity coefficient (%) |
|---|---|---|---|---|---|---|---|---|---|
| 20 | C 12-MCM-41 | 0.5 | 523 | 45.3 | 25.7 | 0.0 | 0.0 | 64.7 | 1.2 |
| 21 | C12-MCM-41 | 0.5 | 573 | 58.7 | 95.7 | 0.1 | 0.0 | 5.9 | 2.4 |
| 22 | C12-MCM-41 | 0.5 | 623 | 91.5 | 99.0 | 0.3 | 0.0 | 0.5 | 1.9 |
| 23 | C12-MCM-41 | 0.5 | 673 | 100.0 | 99.0 | 0.3 | 0.0 | 0.0 | 1.1 |
| 24 | C12-MCM-41 | 0.5 | 723 | 100.0 | 99.0 | 0.6 | 0.0 | 0.0 | 1.1 |

Example 12 (Preparation of catalyst F).

The procedure was conducted in the same manner as in Example 1 but setting the atomic ratio Si/Ni of silicon contained in the C12-MCM-41 and nickel that was added to be 25. The atomic ratio Si/Ni of the obtained catalyst was 28.3 and the surface area was 950 m$^2$/g.

Example 13.

The effect of the contacting time upon the catalytic activity was examined. The contacting time was controlled by varying the amount of the catalyst and the flow rate. The experimental conditions were the same as those of Example 8 but using the catalyst F as catalyst and flowing the mixed gas at a flow rate of 10 to 300 mL/min. Here, the time in which the starting gas contacts the catalyst is defined to be,

```
(Contacting time)[g - catalyst· sec/cc - starting gas]

= (amount of catalyst)[g]/(flow rate)[mL/min] x 60
```

The results were as shown in Table 7.
Table 7

Table 7

| Experiment No. | Catalyst | Amount of catalyst (g) | Gas flow rate (ml/min) | Contacting time (g-catalyst · sec/cc - starting gas) | Temp. (K) | Ethanol conversion (%) | Ethylene selectivity coefficient (%) | Propylene selectivity coefficient (%) | Butene selectivity coefficient (%) | Diethyl ether selectivity coefficient (%) | Acetaldehyde selectivity coefficient (%) |
|---|---|---|---|---|---|---|---|---|---|---|---|
| 25 | F | 0.1 | 300 | 0.02 | 673 | 50.9 | 49.9 | 3.8 | 5.1 | 10.2 | 37.9 |
| 26 | F | 0.2 | 100 | 0.12 | 673 | 97 | 61.2 | 8.3 | 8.2 | 0.8 | 22.7 |
| 27 | F | 0.2 | 50 | 0.24 | 673 | 92 | 67.9 | 10.8 | 5.2 | 0 | 13.7 |
| 28 | F | 0.05 | 10 | 0.30 | 673 | 100 | 75.7 | 12.8 | 8.4 | 0.0 | 1.1 |
| 29 | F | 0.2 | 25 | 0.48 | 673 | 100 | 70.1 | 15.0 | 10.2 | 0.0 | 0.9 |
| 30 | F | 0.1 | 10 | 0.60 | 673 | 100 | 73.8 | 17.9 | 9.6 | 0.0 | 0.9 |
| 31 | F | 0.2 | 10 | 1.2 | 673 | 100 | 69.3 | 24.2 | 11.0 | 0.0 | 0.4 |
| 32 | F | 0.4 | 10 | 2.4 | 673 | 100 | 57.7 | 28.2 | 10.8 | 0.0 | 0.1 |

As the contacting time increased, the acetaldehyde selectivity coefficient and the diethyl ether selectivity coefficient decreased while the propylene selectivity coefficient and the butene selectivity coefficient increased. On the other hand, the ethylene selectivity coefficient was a maximum near 0.3 to 0.6 g - catalyst· sec/cc - starting gas. This was because as the contacting time has increased, the acetaldehyde and diethyl ether have converted into the ethylene. As the contacting time further increased, the ethylene was successively converted into the propylene via the butene.

Example 14.

The effect of the amount of water on the catalytic activity was examined. The experimental conditions were the same as those of Example 8 but adding water in an amount of 0 to 1.4% by volume into the starting gas and using the catalyst in an amount of 0.2 g. The amount of water that is fed is defined to be,

```
(Amount of water that is fed)[vol%] = (gaseous volume of

water)/(total gaseous volume of ethyl alcohol, nitrogen


and water) x 100
```

The gaseous volume was the one found by calculating the weight of water and the like as the volume under the normal pressure. The results were as shown in Table 8.

Table 8

Table 8

| Experiment No. | Catalyst | Amount of catalyst (g) | Temp. (K) | Amount of water (vol%) | Ethanol conversion (%) | Ethylene selectivity coefficient (%) | Propylene selectivity coefficient (%) | Butene selectivity coefficient (%) | Diethyl ether selectivity coefficient (%) | Acetaldehyde selectivity coefficient (%) |
|---|---|---|---|---|---|---|---|---|---|---|
| 33 | C | 0.2 | 673 | 0 | 100 | 66.7 | 15.5 | 5.3 | 0 | 5.5 |
| 34 | C | 0.2 | 673 | 0.3 | 100 | 70 | 16 | 5.2 | 0 | 4.8 |
| 35 | C | 0.2 | 673 | 1.4 | 100 | 71.5 | 17 | 5 | 0 | 4.8 |

Even when water was present in the reaction system, there was almost no change in the ethanol conversion, ethylene selectivity coefficient or propylene selectivity coefficient. This fact teaches that crude bioethanol containing considerable amounts of water can be used as starting material for the reaction.

**Claims**

1. Catalyst obtained by supporting one or two or more kinds of those selected from the group consisting of nickel, aluminum, manganese, iron and copper on regular meso-porous material or comprising the regular meso-porous material, and works to form one or two or more kinds of olefins by using one or two or more kinds of alcohols as starting materials.

2. The catalyst as set forth in claim 1, wherein a main component of the wall constituting the regular meso-porous material is silica.

3. The catalyst as set forth in claim 1, wherein the regular meso-porous material has a pore diameter in a range of 1.4 to 10 nm.

4. The catalyst as set forth in claim 1, wherein one or two or more kinds of those selected from the group consisting of nickel, aluminum, manganese, iron and copper are supported on the regular meso-porous material by a template ion-exchange method.

5. The catalyst as set forth in claim 1, wherein nickel is supported on the regular meso-porous material.

6. The catalyst as set forth in claim 1, wherein the alcohol has carbon atoms in a number in a range of 2 to 10.

7. The catalyst as set forth in claim 1, wherein the alcohol is ethyl alcohol.

8. A method of producing olefins comprising forming one or two or more kinds of olefins by using one or two or more kinds of alcohols as starting materials in the presence of catalyst obtained by supporting one or two or more kinds of those selected from the group consisting of nickel, aluminum, manganese, iron and copper on regular meso-porous material or comprising the regular meso-porous material.

9. The method of producing olefins as set forth in claim 8, wherein a main component of the wall constituting the regular meso-porous material is silica.

10. The method of producing olefins as set forth in claim 8, wherein the regular meso-porous material has a pore diameter in a range of 1.4 to 10 nm.

11. The method of producing olefins as set forth in claim 8, wherein one or two or more kinds of those selected from the group consisting of nickel, aluminum, manganese, iron and copper are supported on the regular meso-porous material by a template ion-exchange method.

12. The method of producing olefins as set forth in claim 8, wherein nickel is supported on the regular meso-porous material.

13. The method of producing olefins as set forth in claim 8, wherein the alcohol has carbon atoms in a number in a range of 2 to 10.

14. The method of producing olefins as set forth in claim 8, wherein the alcohol is ethyl alcohol.

## INTERNATIONAL SEARCH REPORT

| | International application No. |
|---|---|
| | PCT/JP2007/050656 |

**A. CLASSIFICATION OF SUBJECT MATTER**
$B01J29/035$(2006.01)i, $C07C1/24$(2006.01)i, $C07C11/04$(2006.01)i, $C07C11/06$
(2006.01)i, $C07C11/08$(2006.01)i, $C07B61/00$(2006.01)n

According to International Patent Classification (IPC) or to both national classification and IPC

**B. FIELDS SEARCHED**

Minimum documentation searched (classification system followed by classification symbols)
B01J29/035, C07C1/24, C07C11/04, C07C11/06, C07C11/08, C07B61/00

Documentation searched other than minimum documentation to the extent that such documents are included in the fields searched
Jitsuyo Shinan Koho             1922-1996    Jitsuyo Shinan Toroku Koho    1996-2007
Kokai Jitsuyo Shinan Koho      1971-2007    Toroku Jitsuyo Shinan Koho    1994-2007

Electronic data base consulted during the international search (name of data base and, where practicable, search terms used)
JST7580(JDream2), JSTPlus(JDream2)

**C. DOCUMENTS CONSIDERED TO BE RELEVANT**

| Category* | Citation of document, with indication, where appropriate, of the relevant passages | Relevant to claim No. |
|---|---|---|
| X<br><br>Y | JP 56-139425 A  (Shell International Research Maatschappij B.V.),<br>30 October, 1981 (30.10.81),<br>Claim 1; page 2, upper right column, line 10; page 2, lower right column; line 13 to page 3, upper left column, line 4; page 3, upper right column, line 3 to lower left column, line 1<br>& US 4403044 A        & EP 0035807 A1 | 1-2,5-9,<br>12-14<br>3-4,10-11 |
| X<br>Y | JP 60-248630 A  (Director General, Agency of Industrial Science and Technology),<br>09 December, 1985 (09.12.85),<br>Claim 2; page 4, upper left column, lines 13 to 17, referential examples 1 to 2, 13 to 14<br>(Family: none) | 1-2,8-9<br>3-4,10-11 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>07 February, 2007 (07.02.07) | Date of mailing of the international search report<br>20 February, 2007 (20.02.07) |
|---|---|
| Name and mailing address of the ISA/<br>Japanese Patent Office | Authorized officer |
| Facsimile No. | Telephone No. |

Form PCT/ISA/210 (second sheet) (April 2005)

## INTERNATIONAL SEARCH REPORT

| International application No. |
|---|
| PCT/JP2007/050656 |

C (Continuation).   DOCUMENTS CONSIDERED TO BE RELEVANT

| Category* | Citation of document, with indication, where appropriate, of the relevant passages | Relevant to claim No. |
|---|---|---|
| Y | Maki Yonemitsu, Yasuhiro Tanaka, Masakazu Iwamoto, Metal Ion-Planted MCM-41. 1. Planting of Manganese(2) Ion into MCM-41 by a Newly Developed Template-Ion Exchange Method, Chemistry of Materials, 1997.12, Vol.9, No.12, p.2679-2681 | 3-4,10-11 |
| P,X | Koji KASAI, Teruki HAISHI, Takashi YAMAMOTO, Masakazu IWAMOTO, "Ni Tanji Mesoporous Silica ni yoru Bioethanol no Sentakuteki Teikyu Olefin-ka", Dai 98 Kai Shokubai Toronkai Toronkai A Yokoshu, 26 September, 2006 (26.09.06), page 353 | 1-14 |
| P,X P,A | Teruki HAISHI, Takashi YAMAMOTO, Masakazu IWAMOTO, "(3D4-08) Bioethanol no Yuko Riyo -Ni-MCM-41 ni yoru Teikyu Olefin eno Tenkan", CSJ: The Chemical Society of Japan Dai 86 Shunki Nenkai Koen Yokoshu, 13 March, 2006 (13.03.06), page 186 | 1-3,5-10, 12-14 4,11 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11217343 A
- JP 2003326169 A
- JP 2005023420 A

**Non-patent literature cited in the description**

- **C.W. INGRAM ; R.J. LANCASHIRE.** *Catal. Lett.,* 1995, vol. 31, 395
- **A.K. TALUKDAR ; K.G. BHATTACHARYYA ; S. SIVASANKAR.** *Appl. CataL. A,* 1997, vol. 148, 357
- **A.T. AGUAYO ; A.G. GAYUBO ; A.M. TARRIO ; A. ATUTXA ; J. BILBAO.** *J. Chem. Tech. Biotech.,* 2002, vol. 77, 211
- **K. KASAI ; T. HAISHI ; T. YAMAMOTO ; M. IWAMOTO.** *98th Forum on Catalysts,* 2006, 3105
- **K. KASAI ; T. HAISHI ; T. YAMAMOTO ; M. IWAMOTO.** *36th Forum on Petroleum and Petrochemistry,* 2006, vol. 2A19